# EUROPEAN PATENT SPECIFICATION

(11) **EP 0 579 661 B1**
(45) Date of publication and mention of the grant of the patent: **05.01.2000**
(21) Application number: 92907955.6
(22) Date of filing: 09.04.1992
(51) Int. Cl.: C07K 7/02, G01N 33/68

(54) **BRANCHED PEPTIDES IN ASSAYS FOR ANTIBODIES**
VERZWEIGTE PEPTIDE ZUM NACHWEIS VON ANTIKÖRPERN
PEPTIDES RAMIFIES UTILISES DANS DES ANALYSES D'ANTICORPS

(30) Priority: 09.04.1991 GB 9107434
(43) Date of publication of application: 26.01.1994
(60) Divisional application: 99104216.9
(73) Proprietor: MEDICAL RESEARCH COUNCIL, London W1N 4AL (GB)
(72) Inventor: MARSDEN, Howard, Sinkinson Briarwood, Helensburgh G84 7AP (GB); SUBAK-SHARPE, John, Herbert, Glasgow G12 9NH (GB)
(74) Representative: Harding, Charles Thomas
(86) International application number: GB9200632
(87) International publication number: WO9218528

(56) References cited:
- EP-A- 0 339 695
- JP-A- 2 209 890
- PROCEEDINGS NATIONAL ACADEMY OF SCIENCES USA vol. 85, 1988, WASHINGTON pages 5409 - 55413; TAM , J.P.: 'Synthetic peptide vaccine design: Synthesis and properties of a high-density multiple antigenic peptide system'
- JOURNAL OF IMMUNOLOGICAL METHODS vol. 124, 1989, AMSTERDAM pages 53 - 61; TAM ET AL: 'Multiple antigen peptide'
- CHEMICAL ABSTRACTS, vol. 114, 1991, Columbus, Ohio, US; abstract no. 247789P, TANIHARA ET AL: 'Preparation of peptides for adsorption of antibodies to nicotinic acetylcholine receptor' page 818 ;column 2 ;
- CHEMICAL ABSTRACTS, vol. 94, 1981, Columbus, Ohio, US; abstract no. 26977Q, KOMAN ET AL: 'Bifunctional enkephalin analogs for affinity separation purposes' page 244 ;column 2 ;
- JOURNAL OF IMMUNOLOGICAL METHODS vol. 147, 1992, AMSTERDAM pages 65 - 72; MARSDEN ET AL: 'Advantages of branched peptides in serodiagnosis'

### SEQUENCE LISTING

(1) GENERAL INFORMATION:
   (i) APPLICANT: Inventors
   (ii) TITLE OF INVENTION: Peptide-based Assay
   (iii) NUMBER OF SEQUENCES: 4
   (iv) CORRESPONDENCE ADDRESS:
      (A) ADDRESSEE:
      (B) STREET:
      (C) CITY:
      (D) STATE:
      (E) COUNTRY: USA
      (F) ZIP:
   (v) COMPUTER READABLE FORM:
      (A) MEDIUM TYPE: Diskette, 5.25 inch, Kb storage
      (B) COMPUTER:
      (C) OPERATING SYSTEM:
      (D) SOFTWARE:
   (vi) CURRENT APPLICATION DATA:
   (vii) PRIOR APPLICATION DATA:
      (A) APPLICATION NUMBER:
      (B) FILING DATE:
   (viii) ATTORNEY/AGENT INFORMATION:
      (A) NAME:
      (B) REGISTRATION NUMBER:
      (C) REFERENCE/DOCKET NUMBER:
   (ix) TELECOMMUNICATION INFORMATION:
      (A) TELEPHONE: (B) TELEFAX:
(2) INFORMATION FOR SEQ ID NO: 1:
   (i) SEQUENCE CHARACTERISTICS:
      (A) LENGTH: 7 amino acids
      (B) TYPE: amino acid
      (C) TOPOLOGY: linear
   (ii) MOLECULE TYPE: peptide
   (v) FRAGMENT TYPE: internal fragment
   (ix) FEATURE:
      (D) OTHER INFORMATION: the aa sequence is present (a) in linear peptides and (b) in each of 8 branches of a branched peptide of formula ([(aa sequence)(Gly)₅Lys]₂Lys)₂ Lys Ala
   (xi) SEQUENCE DESCRIPTION: SEQ ID NO: 1:
   Gly Asp Pro Glu Asp Leu Asp
   1 5
(3) INFORMATION FOR SEQ ID NO: 2:
   (i) SEQUENCE CHARACTERISTICS:
      (A) LENGTH: 12 amino acids
      (B) TYPE: amino acid
      (C) TOPOLOGY: linear
   (ii) MOLECULE TYPE: peptide
   (v) FRAGMENT TYPE: internal fragment
   (ix) FEATURE:
      (D) OTHER INFORMATION: the sequence is present (a) in linear peptides and (b) in each of 8 branches of a branched peptide of molecular formula (sequence)₈-(lysine)₇-alanine
   (xi) SEQUENCE DESCRIPTION: SEQ ID NO: 2:
   Asp Leu Gly Gln Glu Ser Ala Phe Glu Lys Glu Tyr
   1 5 10
(4) INFORMATION FOR SEQ ID NO: 3:
   (i) SEQUENCE CHARACTERISTICS:
      (A) LENGTH: 17 amino acids
      (B) TYPE: amino acid
      (C) TOPOLOGY: linear
   (ii) MOLECULE TYPE: peptide
   (v) FRAGMENT TYPE: internal fragment
   (ix) FEATURE:
      (D) OTHER INFORMATION: the sequence is present in linear peptides
   (xi) SEQUENCE DESCRIPTION: SEQ ID NO: 3:
   Val Ser His Gly Asp Pro Glu Asp Leu Asp Gly Ala Ala Arg Ala Gly
   1 5 10 15
   Glu
(5) INFORMATION FOR SEQ ID NO: 4:
   (i) SEQUENCE CHARACTERISTICS:
      (A) LENGTH: 13 amino acids
      (B) TYPE: amino acid
      (C) TOPOLOGY: linear
   (ii) MOLECULE TYPE: peptide
   (v) FRAGMENT TYPE: internal fragment
   (ix) FEATURE:
      (D) OTHER INFORMATION: the aa sequence is present (a) in linear peptides and (b) in each of 8 branches of a branched peptide of formula ([(aa sequence)(Gly₀ or 4 Lys]₂Lys)₂ Ala
   (xi) SEQUENCE DESCRIPTION: SEQ ID NO: 4:

## Description

### Background of the invention

### 1. Field of the invention

This invention relates to a method of assay in which a peptide is used for the detection of antibody.

### 2. Description of the related art

Synthetic peptides corresponding to short linear stretches of protein sequences can be recognised by some antibodies directed against the whole protein. They are attractive reagents for detection of serum antibodies directed against such proteins because they are relatively easy to synthesise and are generally cheaper to produce than the whole protein. Peptide-based ELISA kits can be sensitive and specific for the diagnosis of HIV antibodies in AIDS patients, A. Närvänen et al., J. Med. Virology 26, 111-118 (1988), and are now commercially available.

Synthetic peptides have also been used to generate antisera reactive with proteins of which the peptide constitutes a linear part, B. Walter et al., Proc. Natl. Acad. Sci. USA 77, 5197-5200 (1980) and J. C. Sutcliffe et al., Nature 287, 801-805 (1980). More recently, branched peptides have been used to raise anti-peptide antisera which are reactive with proteins, J. P. Tam, Proc. Natl. Acad. Sci. USA 85, 5409-5413 (1988); D. N. Possnett et al., J. Biol. Chem. 263, 1719-1725 (1988). These branched peptides consist of a small two-fold bifurcating polylysine core onto which the peptide of interest is synthesised. It has been shown that such branched peptides are highly immunogenic and elicit a greater immune response in mice than do the corresponding linear (monomeric) peptides, G. Del Guidice et al., Eur. J. Immunol., 20, 1619-1622 (1990) and also that the use of branched peptides in immunoassays give excellent immunoreactivity, increasing the sensitivity and reliability of these assays, J. P. Tam and F. Zavala, J. Immunological Methods, 124, 53-61 (1989), thus enabling lower concentrations of antigen to be used to detect antibodies.

The above-cited 1988 paper of Tam employs ELISA to check that the branched peptides are immunogenic. Thus, under the heading "Test of the MAP to Elicit Specific Antisera", it describes reacting identified branched peptides, having peptides residues of 9-16 amino acids borne on an 8-valent lysine core matrix, with a polyclonal antibody raised against the branched peptide.

It also describes a competitive ELISA in which the "monomeric" (unbranched) peptide competes with the branched peptide for a limited amount of the antibody. Another part of the same paper, headed "Concept and Design" describes the branched peptides as having a triglycyl linker or extender joining peptides of 6-15 amino acid residues to the core matrix (branched carrier) having 3-15 trifunctional amino acids.

The above-cited 1989 paper of Tam *et al.,* received by the journal in March 1989 and therefore after publication of the 1988 paper, refers to the use of branched peptides in immunoassays, but, here, no linkers are referred to, although in retrospect a 17-residue peptide (Asp Pro Pro Pro Pro Asn Pro Asn)₂Gly could be considered to consist of a 16-residue epitopic peptide and a single glycine linker.

However, the use of synthetic peptides as reagents in binding assays such as ELISA is at present limited by their high cost.

### Summary of the invention

It has now surprisingly been found that branched peptides which contain a spacer arm of length equivalent to from 4 to 20 glycine residues between the epitope portion and the polyfunctional core have a marked advantage over the corresponding monomeric peptides and over branched peptides lacking such a spacer arm, in a binding assay, since weight for weight they give rise to a remarkably greater sensitivity of assay.

Although branched peptides have been described as highly immunogenic and as useful in generating antibodies against the corresponding linear peptide in the above-cited papers, and although they have been used in ELISAs against antibodies raised against peptides, it was most unexpected to find that the addition of a spacer arm to the branched structure in between the epitope containing portion and the polyfunctional core would increase the immunogenicity to such an extent that they could be used in assays for the detection of antibodies in very much lower amounts than their linear counterparts, or the branched structures wherein the epitope-containing portion was directly attached to the core without a spacer arm.

The invention provides a method of assay of the body fluid (preferably serum) of a patient, for the presence therein of antibodies, in which a binding reaction is performed between the antibody and a peptide having an epitope therefor and the occurrence or extent of binding is detected or measured respectively, characterised in that the peptide has a branched molecular structure wherein epitope portions are attached to spacer arms, comprising amino acid residues, projecting out of a polyfunctional core, thus forming branches presenting a plurality of such epitopes within the same molecule, located on its branches, each spacer arm being of equivalent length to that provided by from 4 to 20 residues of glycine.

The invention further provides the use of the branched peptides of the invention in such a binding assay and a kit comprising the branched peptide of the invention together with at least one other essential component for the carrying out of or standardisation of such an assay, particularly at least one reagent for the detection or measurement of the binding, e.g. a labelled anti-human immunoglobulin antibody.

### Brief description of the drawings

Figures 1, 2 and 3 are graphs showing the reactivity of branched peptides containing spacer arms of different lengths, compared with monomeric peptides and branched peptides without spacer arms.

Figure 1 shows the effect of spacer arms consisting of glycine residues on the ability of branched peptides to detect anti-peptide antibodies.

Figures 2 and 3 show the effect of spacer arms consisting of glycine residues on the ability of branched peptides to detect anti-peptide antibody. The amount of branched peptide in each of the wells of the assay plate is 1µg (Figure 2) or 0.01µg (Figure 3).

Figures 4,5 and 6 are graphs showing the reactivity of branched peptides containing spacer arms of 4 glycine residues compared with monomeric peptides and branched peptides without spacer arms. The peptide was an HIV epitope.

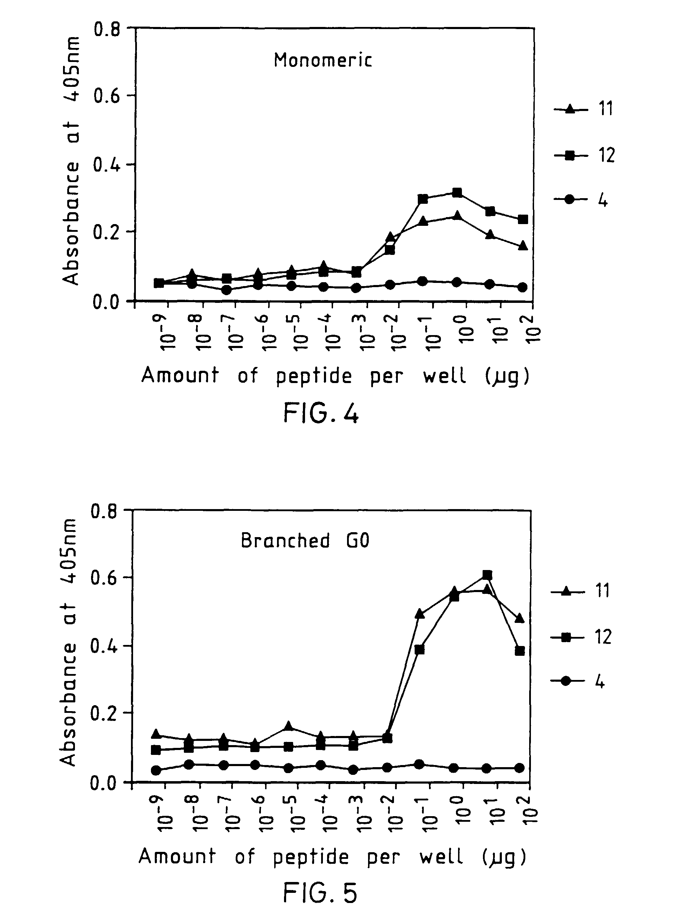

Figure 4 shows the ability of the monomeric peptide to detect anti-peptide antibody at different concentrations of the peptide.

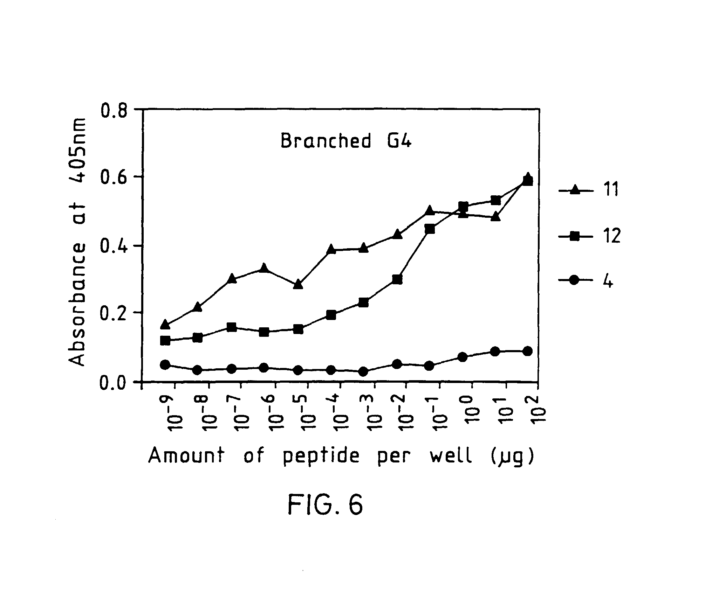

Figures 5 and 6 show the ability of branched peptides with and without spacer arms to detect anti-peptide antibody at different concentrations of peptide.

### Description of the preferred embodiments

The term "epitope" as used herein means all that portion of a protein which interacts with an antibody. The term "spacer arm" as used herein means the residue or residues lying intermediate to the end of the epitope nearest to the core and the outermost branching point of the core, but, where this branching point is provided by the residue of a polyfunctional amino acid, excluding this amino acid residue from consideration. The peptide may include, in addition to the "epitope portion", residues beyond the end of the epitope furthest from the core.

The branched peptides of the invention can be any branched molecule in which the same peptide epitope occurs more than once in the molecule and is present on more than one branch. Although, as the term "peptide" implies, the molecule is predominantly made up of peptide chains and is a non-natural molecule, it is not necessary that the entire molecule be composed of amino acids. A non-peptide core or partly non-peptide spacer or non-peptide linkages between one branch and another can be included.

The spacer arms radiate outwardly from the central core of the molecule and comprise residues of amino acids. Preferably the spacer arms comprise any non-bulky and non-hydrophobic molecular residue. The term "non-polar amino acid" as used herein includes alanine, valine, leucine and isoleucine. Preferably, the spacer arm comprises a majority of residues of glycine (which is an uncharged polar amino acid) or non-polar amino acids. The preferred length of the spacer arm is at least 4 amino acid residues, but even a single residue has a small effect. Little advantage is likely in having more than 20 residues as there may be too much possibility of interference between such highly flexible branches, one with another. Particularly preferred are lengths equivalent to that provided by between 4 and 10 residues of glycine, and most preferably between 4 and 7 residues, where the particularly preferred length is equivalent to that provided by 5 residues of glycine.

The peptide of the invention thus contains branches which can radiate outwardly from any central core of a molecule. The central core and the spacer arms are not so excessively large as to interfere with the assay and otherwise fulfil the requirements of an assay directed to antibodies. Thus, since in most cases the peptide will be caused to adhere to a solid surface such as that of a microtitre plate, dipstick or beads, the core and spacer arms must not interfere with this function.

The core preferably itself comprises a polyfunctional amino acid or a polymer thereof, but could in principle be built up from a simple polyfunctional molecule such as a polyol, e.g. trimethyolpropane, or a polyamine, or from a branched oligomer such as a low molecular weight cross-linked polyacrylate or polymethacrylate. Most preferably, the core is built-up by successive outwards branching and consists of or includes the residue of a polymeric molecule derived from xⁿ -1 molecules of a monomer having a polyfunctionality x, x being at least 2 and usually from 2 to 4, and n being an integer. While n has no upper limit of principle, it is likely that the peptide would have to be chemically synthesised and as a matter of convenience, therefore, n would probably rarely exceed 10 and even more rarely 20.

In the interests of achieving the desired effect to a highly worthwhile degree, n is preferably at least 3; thus, for example, if x is 2 and n is 3, the peptide would be a branched octamer. The monomer is preferably a basic amino acid such as lysine.

The polyfunctional core will normally be fully "saturated" by branches of spacer arms, preferably comprising amino acid residues, extending from the functional groups, usually by peptidic bonds between amine functional groups of the core and carboxyl ends of the spacer arms residues. Advantageously, all epitopes are attached to the core via spacer arms, although if diminishing advantages are acceptable some could be attached direct to the core. By definition, all the spacer arms are attached to epitope-containing portions. This will usually be by peptidic bonds between the amine groups of the spacer arm residues and the carboxyl ends of the epitope-containing chains. A single spacer arm can carry thereon one epitope or a plurality of epitopes, but preferably not more than 2 or 3 per arm.

In principle, however, the branched peptides could be prepared to have some spare arms equivalent to spacer arms but with no epitope attached. Again, there would normally be diminishing advantage in such a construction.

Generally stated, the branched peptide molecule of the invention will contain only one kind of epitope for binding to antibodies specific thereto. Of course, if there are two or more kinds of antibodies to be detected in a single assay, e.g. if an illness could be caused by two or more infective agents lacking convenient common epitopes and if the illness is treated in a same way whichever infective agent is responsible, the peptide could incorporate different epitopes. Alternatively, the assay could be carried out using separate branched peptides of the invention each containing a single epitope. In order to avoid the possibility of false positives, the peptide will not contain any linkages which could give a binding reaction with other antibodies or a non-specific binding reaction. It will therefore not contain segments of any proteins which might produce such effects.

The peptides can be made by any of the well known synthetic methods, e.g. as described by Tam or by Posnett et al. cited above, but the Fmoc method, e.g. as described by H. Lankinen et al., J. Gen. Virol. 70, 3159-3169 (1989) is particularly convenient.

The invention can be practised in any way appropriate to the detection of antibodies, especially on any appropriate fluid, especially a body fluid, likely to contain antibodies. Although serum is the usual body fluid, other fluids which contain certain immunoglobulins, e.g. tears, saliva, or milk, could be assayed.

The invention enables small amounts of peptide to be used, the amount of branched peptide of the invention required being ordinarily less than of its linear counterpart or branched counterpart wherein the branched counterpart does not contain a spacer arm. The comparison is reckoned on the total weight of branched peptide inclusive of core and spacer arm against the total weight of the linear peptide of the same or substantially the same amino acid composition. The comparison can be made either in terms of the amount of peptide required to achieve a given level of signal or in terms of the concentration of antibody in serum which can be detected by use of a given amount of the peptide.

In normal practice, the assay will be carried out heterogeneously by attaching the branched peptide of the invention to an insoluble carrier such as the plastic surface of a microtitre plate, although the invention is not limited to such assays. In a sandwich assay, the antiserum is incubated with the peptide for an appropriate period to allow binding and with an antibody to the immunoglobulin. Thus, for example, if the patient is a human and the antibody is of the IgG class, an anti-human IgG antibody, appropriately labelled, is added, the plate is washed and the amount of label bound to the plate (via the antibody and the peptide) is measured. Alternatively, a competition assay can be performed in which a known amount of labelled antibody is permitted to compete with the serum or to displace it from its binding. Such an assay, however, is less appropriate for detecting small concentrations of antibody. Any of the usual labels, e.g. radiolabels and enzyme labels, can be used, especially peroxidase and alkaline phosphatase. Radio labels can be assayed by radioactive counting and enzyme labels by a colorimetric, fluorescent or chemiluminescent signal, for example. An amplification method of detection, such as "Ampak" (Registered Trade Mark) of Nordisk Diagnostics Ltd., which requires an alkaline phosphatase enzyme label, can be used to further sensitise the assay.

The amount of peptide to be used per assay, e.g. per well of a plate, will normally be less than 5µg, preferably less than 1µg and most preferably less than 0.1µg. The most suitable amount in any particular case will depend on the nature of the epitope involved. In a conventional microtitre plate having a capacity of 100 µl per well, the peptide solution used would then be less than 5mg.

In a kit for carrying out the assay, the essential components will in most cases be the branched peptide of the invention and an anti-human immunoglobulin labelled antibody. These are, of course, provided in the kit in separate containers.

The following Examples illustrate the invention. "Tween" is a Registered Trademark.

### Example 1

### Synthesis of linear and branched peptides

Peptides were synthesised by continuous-flow Fmoc chemistry using an LKB peptide synthesiser and a procedure described by H. Lankinen et al., J. Gen. Virol., 70, 3159-3169 (1989). Following synthesis, side-chain protecting groups were removed by treatment with 95% trifluoroacetic acid in water (plus 4% w/v phenol for the arginine-containing peptides, or 3% w/v phenol plus 2% ethanedithiol for cysteine and arginine containing peptides.

Relative molecular masses of the linear peptides were determined by mass spectrometry (M-Scan Ltd.) which gave values identical to those expected. The amino acid composition of branched peptides was determined by amino acid analysis (Cambridge Research Biochemicals) and again were in agreement with expectations. The purity of peptides was checked by reverse-phase HPLC using a "Dynamax" analytical column (4.6mm internal diameter x 25cm length, 300A C8 resin, Catalogue Number 83-303-C) using a gradient of 0%-95% acetronitrile in water of 20 minutes duration and flow rate of 0.5ml/min. Purities ranged from 61% to 91%.

The peptides synthesised are set out in Table 1 below:

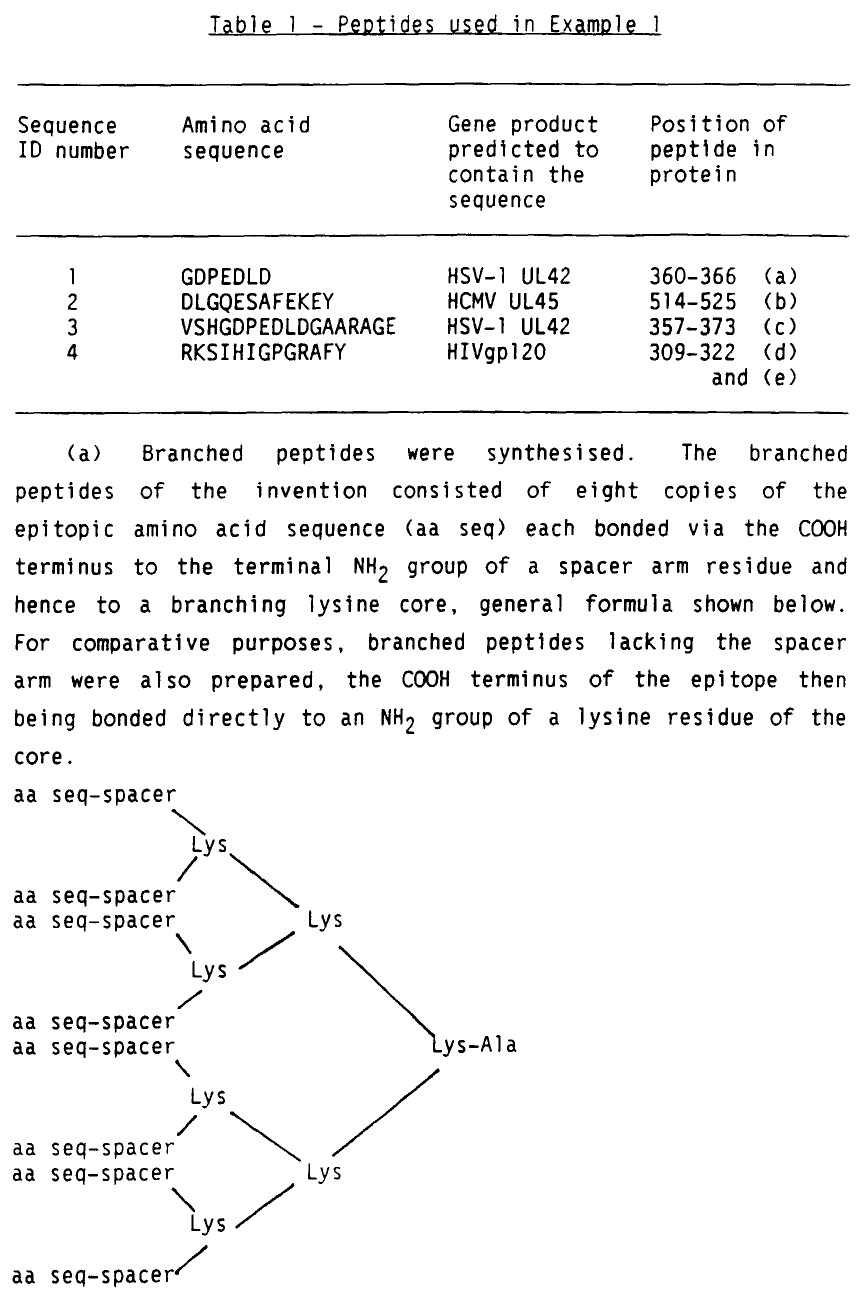

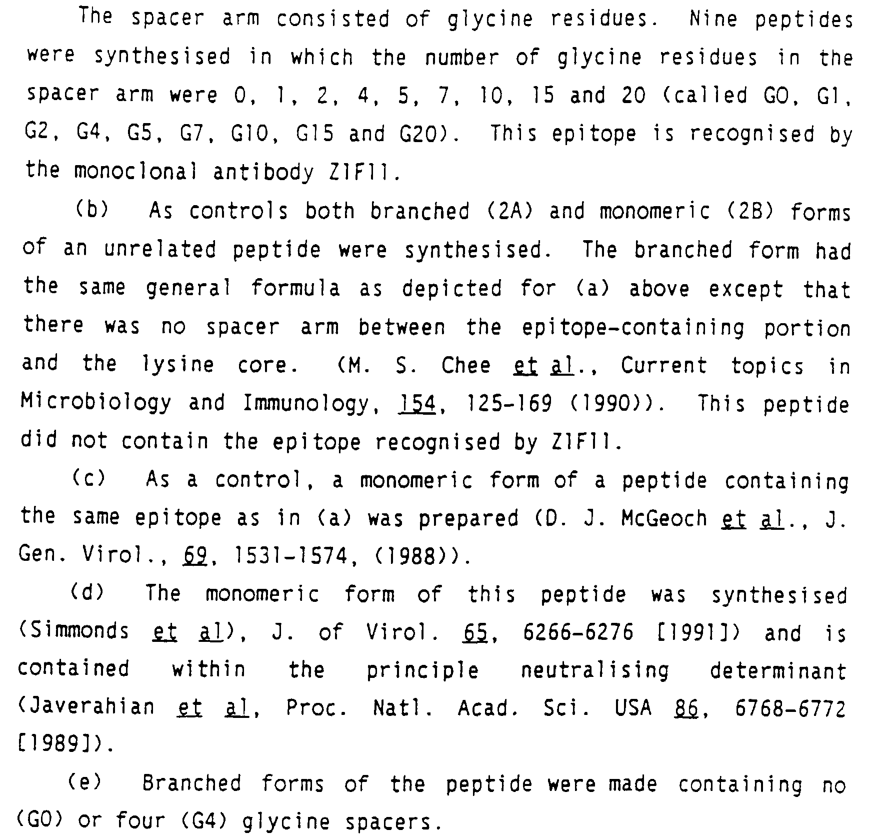

### Example 2

### Enzyme-linked immunosorbent assay (ELISA)

The effect of varying the distance between the reactive epitope and the core on the reactivity of the branched molecule with the detecting antibody was investigated.

Nine peptides (sequence ID number 1) were synthesised as described in Example 1. Monoclonal antibody Z1F11 (P. Schenk et al., J. Gen. Virol., 69, 99-111 (1988)), a monoclonal antibody raised against the herpes simplex virus HSV-I, was employed in the ELISA assay since this had previously been shown to recognise the sequential epitope GDPEDLD (M. Murphy et al., J. Gen. Virol., 70, 2357-2364 (1989)).

### The ELISA was carried out as follows:

Peptides were dissolved in phosphate buffered saline (PBS) at a concentration of 1 mg/ml. The peptides were then diluted in PBS (phosphate buffered saline) to solutions varying in concentration between 1 mg/ml and 1 pg/ml. 100µl of peptide solution was added to each well, representing amounts of peptides ranging from 100µg down to 100fg. The peptides were allowed to adsorb to the plate at 37°C overnight. Excess antigen solution was removed and the plates blocked in a solution containing 1% BSA for lhr at 37°C. The plates were then washed three times in PBS containing 0.01% "Tween-20" (PBS-Tween) and incubated with 100µl of the appropriate antibody dilution at 37°C for lhr. For detection of bound antibodies, plates were washed five times in PBS-Tween and incubated for lhr at 37°C with 100µl per well of horseradish peroxidase-sheep anti-mouse conjugate (SAPU) (diluted 1000-fold in PBS). Plates were then washed seven times in PBS-Tween and reacted with a 50µg/ml solution of the enzyme substrate 2,2'-azino-bis(3-ethylbenzothiazoline-6-sulphonic acid) (ASTS] in citrate phosphate buffer containing 0.01% hydrogen peroxide. After 15 min. of colour change development, the plates were read on a Titerek Multi scan plate reader at 405nm. The results given are all the mean of duplicate determinations.

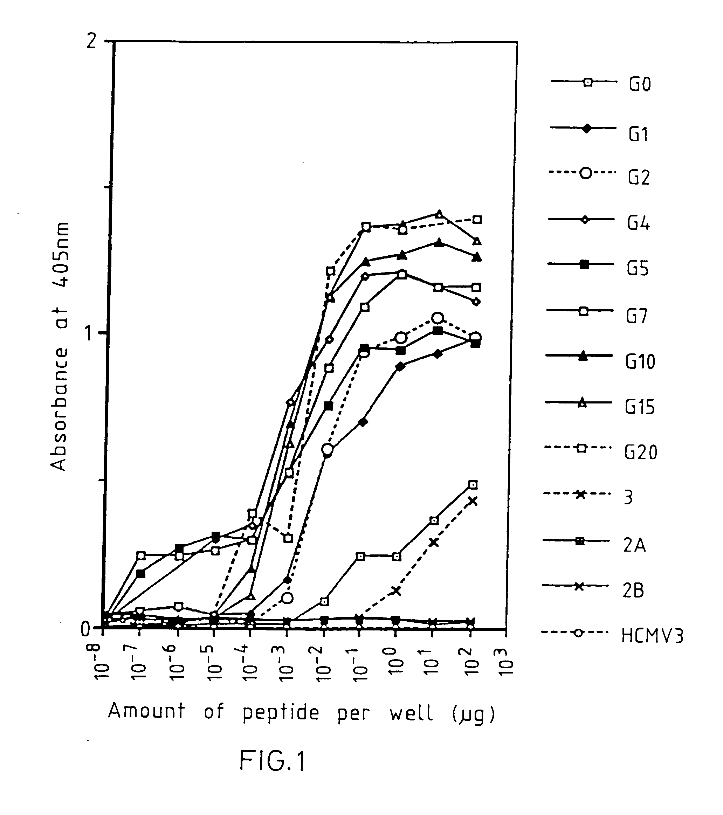

The results of the first experiment are shown in Figure 1, different amounts of peptides, ranging from 100µg down to 100fg were tested in ELISA assays for reactivity with Z1F11 (diluted 1/2000). Controls included: monoclonal antibody HCMV-3, (a monoclonal antibody raised against human cytomegalovirus which would not be expected to react with any of the peptides of sequence ID numbers 1, 2 and 3) in place of Z1F11, peptide 2 (Table 1) in both branched 2A and monomeric 2B forms as described in Example 1 and monomeric peptide sequence ID number 3 (Table 1), which contains the Z1F11-reactive epitope. The figure shows that all branched peptides containing the sequence GDPEDLD reacted more strongly with Z1F11 than did the monomeric peptide containing this sequence (sequence ID number 3). However, there was a marked difference between the branched peptide (sequence ID number 1) with no glycine spacer (G0), which was only marginally more reactive than the monomeric peptide, and the branched peptide with one glycine spacer (G1): at 100µg peptide per well G1 gave twice the absorbance of that produced by G0 and 10⁴-fold more G0 than G1 was needed to produce an absorbance of 0.5. Additional glycine residues progressively increased the absorbance for amounts of peptide about 10pg per well, although peptides G5 and G7 appeared to be the most sensitive at low concentrations. The controls behaved as expected in that firstly, the control antibody HCMV-3 did not react with any peptide at any concentration (only the data for reaction of this antibody with peptide G10 is shown), secondly, Z1F11 did not react with the unrelated peptides sequence ID number 2A (branched) and sequence ID number 2B (monomeric) and thirdly, in the absence of peptide, Z1F11 was not reactive.

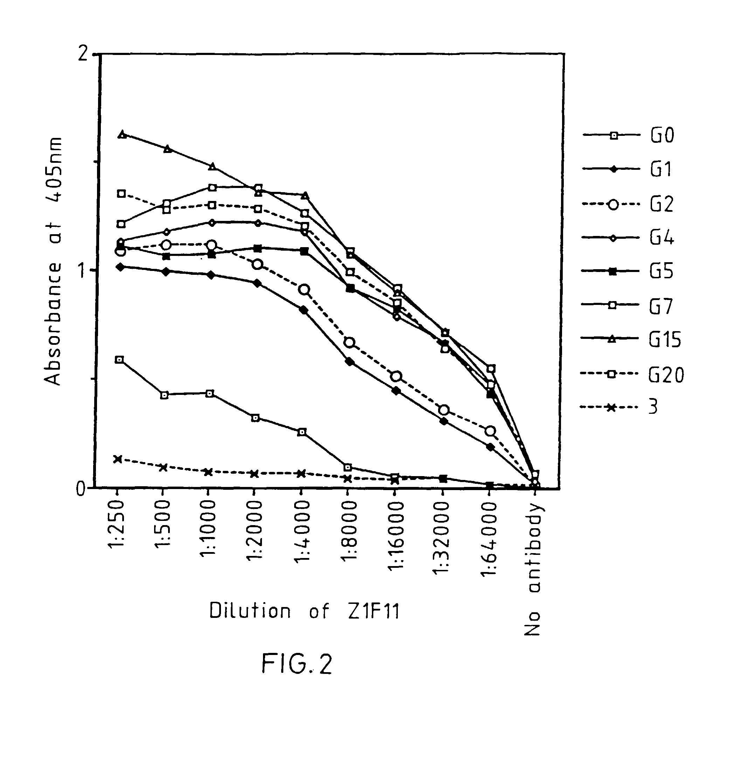

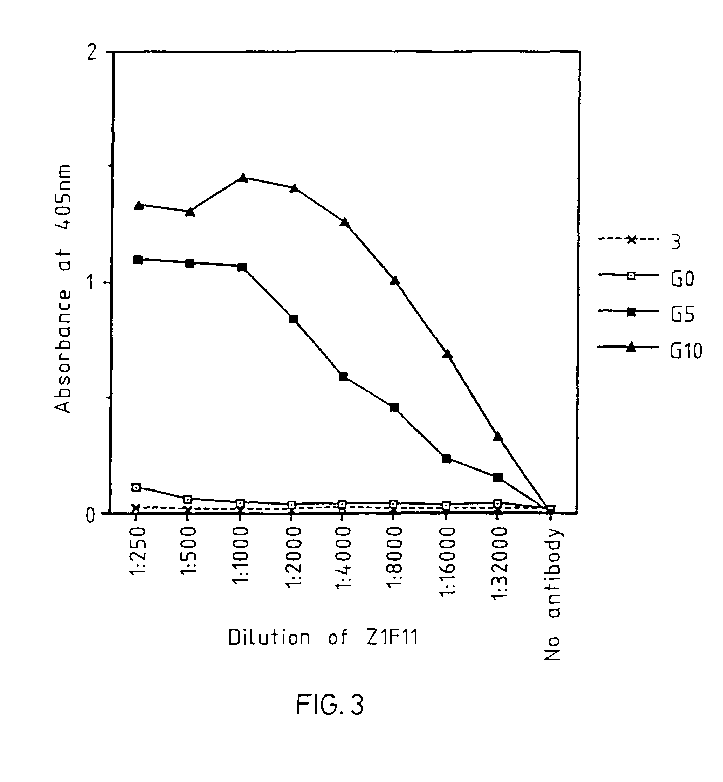

The results of the second experiment are shown in Figures 2 and 3. The effect of the length of the glycine spacer on the sensitivity with which branched peptides (sequence ID number 1) could detect Z1F11 was examined. Three different amounts of peptide were tested: 0.01µg, 1µg and 100µg. All gave essentially the same results and only the data for 1µg per well and 0.01µg per well are presented in Figures 2 and 3 respectively. The reactivity of the peptides was tested against doubling dilutions of Z1F11 ranging from 1/250 to 1/64000. The results for branched peptides used at a concentration of 1µg/well are shown in Figure 2. Here all forms of the branched peptide were significantly more reactive than the control monomeric peptide (sequence ID number 3). The effect was particularly marked with four or more glycine residues. When diluted 64000-fold Z1F11 yielded an absorbance of 0.5 with G4 peptide, whereas to produce the same absorbance with peptide GO the antibody could only be diluted 250-fold. Additional glycine residues did not significantly increase the reactivity of the branched peptide. It is concluded from this experiment that a spacer of four or more glycine residues increases the sensitivity for detection of antibody Z1F11 by at least 256-fold.

The results for peptides 3, G0, G5 and G10 used at 0.01µg/well are shown in Figure 3. At this concentration peptide 3 did not detect Z1F11 at any concentration and the G0 peptide showed only marginal reactivity. In contrast the G5 and G10 showed good reactivity.

### Example 3

### Enzyme-linked immunosorbent assay

The effect of varying the distance between the reactive epitope and the core on the reactivity of the branched molecule with the detecting antibody was investigated. A different epitope to that of Example 2 was used.

Three peptides (sequence ID number 4) were synthesised as described in Example 1 (d) and (e). Human sera designated numbers 11 and 12 were from two HIV-1 antibody positive individuals. Human serum designated number 4 was obtained from an HIV-1 antibody negative individual.

### The ELISA was carried out as follows:

Peptides were dissolved in phosphate buffered saline (PBS) at a concentration of 1 mg/ml. The peptides were then diluted in PBS (phosphate buffered saline) to solutions varying in concentration between 1 mg/ml and 10 fg/ml. 100µl of peptide solution was added to each well, representing amouts of peptides ranging from 50µg down to lfg. The procedure for carrying out the ELISA was exactly as described in Example 2 except that the PBS-Tween solution used for washing the plates was PBS containing 0.05% "Tween-20". The results given are all the mean of duplicate determinations.

The results are shown in Figures 4,5 and 6. Different amounts of peptides, ranging from 50µg down to 1fg, were tested in ELISA assays for reactivity with human sera from the two HIV-1 antibody positive individuals (diluted 1/100). Human sera from an HIV-1 antibody negative individual served as a control.

Figure 4 shows that the monomeric peptides reacted less strongly with HIV-1 antibody positive human sera (numbers 11 and 12) than did the branched peptide containing no glycine spacers (G0) shown in Figure 5 which in turn reacted less well than the branched peptide containing four glycine spacers (G4) shown in Figure 6: neither sera 11 nor 12 were significantly reactive with less than 5×10⁻⁴µg of the G0 branched peptide, whereas they were reactive with at least 1000-fold lower concentrations of the G4 branched peptide.

The following claims define some important aspects of the invention.

## Claims

1. A method of assay of antibodies in a sample of a body fluid obtained from a patient, in which a binding reaction is performed between the antibodies and a peptide having a plurality of an epitope portion therefor and the occurrence or extent of binding is detected or measured respectively, and in which the peptide is a branched peptide comprising the epitope portions attached to spacer arms, comprising amino acid residues, projecting out of a polyfunctional core, thereby presenting a plurality of the epitopes within the same molecule located on its branches, characterised in that each spacer arm is of equivalent length to that provided by from 4 to 20 residues of glycine.

2. A method according to claim 1 characterised in that the spacer arms comprise non-hydrophobic molecular residues.

3. A method according to claim 1 or 2, characterised in that the majority of the amino acid residues within the spacer arms are glycine or non-polar amino acids.

4. A method according to claim 3, characterised in that all the amino acids are glycine.

5. A method according to any preceding claim, characterised in that the spacer arm is of equivalent length to that provided by from 4 to 10 residues of glycine.

6. A method according to claim 5, characterised in that the spacer arm is of equivalent length to that provided by 4 to 7 residues of glycine.

7. A method according to any preceding claim, characterised in that the core comprises a residue of a polymeric molecule derived from xⁿ-1 molecules, of a polyfunctional monomer, where x is the functionality of the monomer and is at least 2 and n is an integer.

8. A method according to claim 7 characterised in that the monomer is lysine.

9. A method according to any preceding claim, characterised in that the peptide is used in a smaller amount by weight than would be required to achieve the same sensitivity of assay or to produce a given signal if a corresponding linear peptide or a branched peptide lacking a spacer arm between the core and the epitopes were used instead.

10. A method according to any preceding claim, characterised in that the peptide is used in an amount of less than 5µg/well.

11. A method according to claim 10, characterised in that the peptide is used in an amount of less than 1µg/well.

12. A method according to Claim 11, characterised in that the peptide is used in an amount of less than 0.1µg/well.

13. A method according to any preceding claim, characterised in that it takes the form of a sandwich assay.

14. A method according to claim 13, characterised in that the body fluid is serum.

15. A kit for carrying out an assay of a sample of body fluid of a patient for the presence therein of antibodies, in which a binding reaction is performed between the antibodies and a peptide having an epitope therefor, and the occurrence or extent of binding is detected or measured respectively, characterised in that the kit comprises (1) a branched peptide defined in any one of claims 1 to 8; and (2) a labelled anti-human immunoglobulin antibody for detection or measurement of said binding.

## Patentansprüche

1. Antikörper-Assayverfahren in einer Probe einer Körperflüssigkeit, die von einem Patienten erhalten wurde, indem man eine Bindungsreaktion zwischen den Antikörpern und einem Peptid mit mehreren Epitopanteilen hierfür ausführt und das Auftreten oder das Ausmaß von Bindung feststellt bzw. mißt, wobei das Peptid ein verzweigtes Peptid ist, das die Epitopanteile an Abstandshalterarme gebunden umfaßt, welche Aminosäurereste umfassen, die von einem polyfunktionellen Kern aus vorspringen, und dabei mehrere der Epitope in dem gleichen Molekül an seinen Verzweigungen liegend aufweist, dadurch gekennzeichnet, daß jeder Abstandshalterarm äquivalente Länge zu der hat, die von 4 bis 20 Glycinresten erhalten wird.

2. Verfahren nach Anspruch 1, dadurch gekennzeichnet, daß die Abstandshalterarme nicht-hydrophobe Molekülreste umfassen.

3. Verfahren nach Anspruch 1 oder 2, dadurch gekennzeichnet, daß der Hauptteil der Aminosäurereste in den Abstandshalterarmen Glycin oder nicht polare Aminosäuren sind.

4. Verfahren nach Anspruch 3, dadurch gekennzeichnet, daß alle Aminosäuren Glycin sind.

5. Verfahren nach einem der vorausgehenden Ansprüche, dadurch gekennzeichnet, daß der Abstandshalterarm von äquivalenter Länger zu jener ist, die von 4 bis 10 Glycinresten gebildet wird.

6. Verfahren nach Anspruch 5, dadurch gekennzeichnet, daß der Abstandshalterarm von äquivalenter Länge zu jener ist, die von 4 bis 7 Glycinresten gebildet wird.

7. Verfahren nach einem der vorausgehenden Ansprüche, dadurch gekennzeichnet, daß der Kern einen Rest eines sich von xⁿ-1 Molekülen herleitenden Polymermoleküls eines polyfunktionellen Monomers umfaßt, worin x die Funktionalität des Monomers und wenigstens 2 ist und n eine ganze Zahl bedeutet.

8. Verfahren nach Anspruch 7, dadurch gekennzeichnet, daß das Monomer Lysin ist.

9. Verfahren nach einem der vorausgehenden Ansprüche, dadurch gekennzeichnet, daß das Peptid in einer kleineren Gewichtsmenge verwendet wird, als erforderlich wäre, die gleiche Assay-Empfindlichkeit zu erreichen oder ein bestimmtes Signal zu erzeugen, wenn ein entsprechendes lineares Peptid oder ein Abstandshalterarm ohne verzweigtes Peptid zwischen dem Kern und den Epitopen stattdessen verwendet würden.

10. Verfahren nach einem der vorausgehenden Ansprüche, dadurch gekennzeichnet, daß das Peptid in einer Menge von weniger als 5 µg/Platz verwendet wird.

11. Verfahren nach Anspruch 10, dadurch gekennzeichnet, daß das Peptid in einer Menge von weniger als 1 µg/Platz verwendet wird.

12. Verfahren nach Anspruch 11, dadurch gekennzeichnet, daß das Peptid in einer Menge von weniger als 0,1 µg/Platz verwendet wird.

13. Verfahren nach einem der vorausgehenden Ansprüche, dadurch gekennzeichnet, daß es in der Form eines Sandwich-Assays stattfindet.

14. Verfahren nach Anspruch 13, dadurch gekennzeichnet, daß die Körperflüssigkeit Serum ist.

15. Kit zur Durchführung eines Assays mit einer Probe einer Körperflüssigkeit eines Patienten hinsichtlich des Vorhandenseins von Antikörpern dann, wobei die Bindungsreaktion zwischen den Antikörpern und einem Peptid mit einem Epitop hierfür durchgeführt wird und das Vorhandensein oder Ausmaß von Bindung festgestellt bzw. gemessen wird, dadurch gekennzeichnet, daß der Kit (1) ein in einem der Ansprüche 1 bis 8 definiertes verzweigtes Peptid und (2) einen markierten Anti-human-lmmunoglobinantikörper zur Feststellung oder Messung der Bindung umfaßt.

## Revendications

1. Méthode d'analyse d'anticorps dans un échantillon d'un liquide biologique obtenu à partir d'un patient, dans laquelle une réaction de liaison est conduite entre les anticorps et un peptide ayant une pluralité de portions épitopiques pour ces anticorps et l'apparition ou le degré de liaison est détecté ou mesuré, respectivement, dans laquelle le peptide est un peptide ramifié comprenant les portions épitopiques fixées à des branches intercalaires, comprenant des résidus d'amino-acides, faisant saillie hors d'un noyau polyfonctionnel, en présentant ainsi une pluralité des épitopes dans la même molécule présents sur ces ramifications, caractérisée en ce que chaque branche intercalaire a une longueur équivalente à celle fournie par 4 à 20 résidus glycine.

2. Méthode suivant la revendication 1, caractérisée en ce que les branches intercalaires comprennent des résidus moléculaires non hydrophobes.

3. Méthode suivant la revendication 1 ou 2, caractérisée en ce que la plupart des résidus d'amino-acides dans les branches intercalaires sont des résidus glycine ou d'amino-acides non polaires.

4. Méthode suivant la revendication 3, caractérisée en ce que tous les amino-acides consistent en glycine.

5. Méthode suivant l'une quelconque des revendications précédentes, caractérisée en ce que la branche intercalaire a une longueur équivalente à celle fournie par 4 à 10 résidus glycine.

6. Méthode suivant la revendication 5, caractérisée en ce que la branche intercalaire est une longueur équivalente à celle fournie par 4 à 7 résidus glycine.

7. Méthode suivant l'une quelconque des revendications précédentes, caractérisée en ce que le noyau comprend un résidu d'une molécule polymérique dérivée de xⁿ-1 molécules d'un monomère polyfonctionnel, x représentant la fonctionnalité du monomère et étant au moins égal à 2 et n représentant un nombre entier.

8. Méthode suivant la revendication 7, caractérisée en ce que le monomère est la lysine.

9. Méthode suivant l'une quelconque des revendications précédentes, caractérisée en ce que le peptide est utilisé en une quantité pondérale inférieure à celle qui serait requise pour parvenir à la même sensibilité de l'analyse ou pour produire un signal donné si un peptide linéaire correspondant ou un peptide ramifié dépourvu de branches intercalaires entre le noyau et les épitopes était utilisé au lieu de ce peptide.

10. Méthode suivant l'une quelconque des revendications précédentes, caractérisée en ce que le peptide est utilisé en une quantité inférieure à 5 µg/puits.

11. Méthode suivant la revendication 10, caractérisée en ce que le peptide est utilisé en une quantité inférieure à 1 µg/puits.

12. Méthode suivant la revendication 11, caractérisée en ce que le peptide est utilisé en une quantité inférieure à 0,1 µg/puits.

13. Méthode suivant l'une quelconque des revendications précédentes, caractérisée en ce qu'elle est sous forme d'une analyse en sandwich.

14. Méthode suivant la revendication 13, caractérisée en ce que le liquide biologique consiste en sérum.

15. Kit pour la mise en oeuvre d'une analyse d'un échantillon d'un liquide biologique d'un patient pour déterminer la présence d'anticorps dans cet échantillon, dans lequel une réaction de liaison est effectuée entre les anticorps et un peptide ayant un épitope pour ces anticorps, et l'apparition ou le degré de liaison est détecté ou mesuré, respectivement, ledit kit étant caractérisé en ce qu'il comprend (1) un peptide ramifié répondant à la définition suivant l'une quelconque des revendications 1 à 8 ; et (2) un anticorps marqué anti-immunoglobuline humaine pour la détection ou la mesure de ladite liaison.
